# EUROPEAN PATENT SPECIFICATION

(11) **EP 2 624 784 B1**
(45) Date of publication and mention of the grant of the patent: **26.01.2022**
(21) Application number: 11767602.3
(22) Date of filing: 03.10.2011
(51) Int. Cl.: A61F 2/16

(54) **INTRAOCULAR LENS IMPLANT**
INTRAOKULARLINSENIMPLANTAT
IMPLANT DE LENTILLE INTRAOCULAIRE

(30) Priority: 06.10.2010 WO PCT/CH2010/000246
(43) Date of publication of application: 14.08.2013
(73) Proprietor: Accommo AG, 8808 Pfäffikon (CH)
(72) Inventor: HAEFLIGER, Eduard Anton, 8808 Pfäffikon (CH)
(74) Representative: Toleti, Martin
(86) International application number: PCT/CH2011/000234
(87) International publication number: WO 2012/045186

(56) References cited:
- EP-A2- 0 337 390
- WO-A1-2004/000171
- WO-A2-02/19949
- WO-A2-2007/075581
- US-A1- 2005 228 401
- US-A1- 2007 260 310
- US-A1- 2008 154 364
- US-A1- 2009 018 652

## Description

### Technical Field

The invention relates to an intraocular lens implant, a method for manufacturing an intraocular lens implant, and a kit for manufacturing an intraocular lens implant.

### Background Art

Replacing the lens of a human eye by means of an intraocular lens implant may be indicated when due to aging processes the natural lens hardens and accommodation may no longer be achievable. For quite a while lens implants allowing accommodation include a replacement of the natural lens mass of the human eye by means of a synthetic lens mass. Besides the requirement for adapting such lens implant according to the individual needs e.g. to a specific refraction index, the materials to manufacture such lens implant from are difficult to elect in view of the diverging needs of the lens implant being accommodatable on the one hand and persistent and longliving on the other hand.

Recently, it was proposed to replace a single body lens implant by a lens implant with two viewing elements, i.e. two lenses being coupled to each other by means of a spring element. Such a lens implant is, for example, referred to in US 2005/0228401 A1. The lens implant comprises an anterior portion including an anterior viewing element and an anterior biasing element. The lens further comprises a posterior portion including a posterior viewing element and a posterior biasing element. The anterior portion and the posterior portion meet at first and second apices of the intraocular lens. The anterior portion and the posterior portion and/or the apices are responsive to force the separation between the viewing elements to change. The lens implant is designed for being implanted into a lens capsule of the eye.

In the absence of any external forces, this lens implant takes a shape in which the viewing elements are at their maximum separation along the optical axis. The viewing elements may be moved towards each other in response to a ciliary muscle relaxation in order to reach a shape corresponding to a state of the lens implant suitable for distant vision, which is also denoted in this document as unaccommodated state. A relaxation of the ciliary muscle makes the zonule fibres become tense and pull the lens capsule radially outwards which invokes a force against the spring element of the lens implant in the lens capsule and compresses the viewing elements. As a result the lens capsule including the lens implant takes a flatter shape. At the other extreme, the ciliary muscle contracts such that the zonule fibres relax. The lens implant and in particular its spring element extends from its tense state into its relaxed state such that the viewing elements are moved away from each other. The lens implant takes a spherical shape corresponding to a state for near vision which is also denoted as accommodated state.

In another embodiment illustrated in the Figures 38A and 38B of the subject document the lens implant provides next to the two viewing elements two biasing elements dimensioned such that their apices abut the zonule fibres and the ciliary muscle when in the unaccommodated state. Here, the lens implant is configured such that it will remain in the unaccommodated state in the absence of external forces. Thus, when the ciliary muscle contracts it pushes the apices closer together causing the biasing elements to bow out and the viewing elements to separate and attain the accommodated state. When the ciliary muscle relaxes, in turn, the force applied to the apices is reduced and the viewing elements approach each other again and convert the lens implant to the unaccommodated state.

Both variants above are not believed to be ideal in terms of adaptability of a lens implant to its natural environment. In the first variant, the relaxed state of the lens implant provides spaced apart viewing elements resulting in a shape representing the accommodated state of the lens implant suited for near vision. This does not reflect the shape of the natural lens mass without its capsule when not being exposed to any external forces: The natural lens mass rather takes a flattened shape representing the unaccommodated state suited for distant vision.

According to the second version, the relaxed state of the lens implant seems to be reversed with respect to the first variant and the shape of the lens implant represents an unaccommodated state of the lens suitable for distant vision. However, the actuation of such lens implant as well as the shape of such lens implant is not considered to be a best fit in terms of adaptability of such lens implant to its environment. One of the reasons is that in the human eye the ciliary muscle typically does not directly drive and engage with the lens.

US 2009/0018652 A1 discloses an intraocular lens for providing accommodative vision to a subject. The lens includes a frame disposed about an optical axis, a first optical element, a second optical element, and a connecting element operably coupling the frame to the optical elements. The frame comprises an anterior frame element and a posterior frame element. The connecting element is configured to convert a first displacement between the frame elements in a direction that is substantially parallel to the optical axis into a second displacement between the optical elements that is substantially perpendicular to the optical axis. The second displacement may be translational and/or rotation. In some embodiments, the optical elements are two varifocal lenses.

### Disclosure of the Invention

Hence, the problem to be solved by the invention is to provide an intraocular lens implant which is adapted to the physiology of the eye, and which is suited for a long term deployment in the lens capsule.

According to an aspect of the present invention an intraocular lens implant is provided comprising a first viewing element, a second viewing element, and a spring element connected to the viewing elements for varying a distance between the first and the second viewing element along an optical axis of the lens implant for varying the focal length of the lens implant. The lens implant is designed to take a shape suitable for distant vision when the spring element is in its relaxed state. A spring constant of the spring element has a value of less than 550 mN/mm. Specifically, the lens implant is designed to take a shape suitable for near vision when the spring element is in a stretched state such that the distance between the first and the second viewing element in the stretched state of the spring element exceeds the distance between the first and the second viewing element in the relaxed state of the spring element. Hence, the lens implant is designed to require an external stretch force which, upon contraction of the ciliary muscle, originates from the lens capsule and acts on the spring element for stretching the spring element from its relaxed state into its stretched state.

Such lens implant is designed to reflect properties of the natural lens and its actuation mechanism as much as possible and respects the physiology of the eye. First, the lens implant although being designed by several components comprising two or more viewing elements and a spring element between the viewing elements is designed to - absent any external forces - take a flattened shape, which shape represents the shape of a natural lens enabling distant vision, i.e. what also sometimes is referred to as the lens being in an unaccommodated state. This shape reflects the shape of the natural lens and as such serves best for any accommodation processes as well as for any other physiological processes.

When the lens implant in its relaxed state takes a shape suitable for distant vision it needs to be convertible from there into a shape suitable for near vision. While in the state of the art this is achieved by members protruding from the viewing elements of the lens implant trying to directly engage with the ciliary muscle such actuation does not reflect the actuation used by the human eye.

For the current lens implant it is envisaged that the ciliary muscle is not itself pushing the lens implant or the lens capsule for the reason that such actuation is not conform with the natural accommodation and may only be achieved by training of the ciliary muscle and the human brain in order to switch to such actuation mechanism different to the one used for the natural eye. Instead, as is with the human eye, upon contraction of the ciliary muscle the zonule fibres relax and no longer stretch the lens capsula which stretching held the lens capsule in a flattened elongated state before.

It was observed that without or with little interaction of the zonule fibres only, it is the lens capsule itself which causes the lens mass transitioning from the flat shape representing distant vision into the more spherical shape representing near vision. The lens capsule is formed by a basement membrane which was built during the growth of the lens mass at its periphery by building subcapsular epithelium cells. The lens capsule encompassing the lens is elastic, and without any other external forces its surface tends to take a shape of lowest surface per volume which is a sphere. This is why absent any external forces the combination of lens mass and lens capsule takes a sphere-like shape which is the desired one for near vision. However, the tension built by the lens capsule needs to overcome the spring force generated by the spring element of the lens implant in a direction for stretching the spring element. Such tension may be between 2 to 50 g/mm in direction of the optical axis subject to the individual. Hence, the spring constant of the spring element may preferably have a value of less than 20 mN/mm. In any case, the spring constant of the spring element has a value of less than 550 mN/mm, and more preferably of equal to or less than 500 mN/mm, and more preferably of less than 300 mN/mm.

In a design step, the spring constant of the spring element of the lens implant is dimensioned such that a force produced by the lens capsule transforms the spring element from its relaxed state into a stretched state upon contraction of the ciliary muscle, i.e. pulls the spring element. The direction of transition is determined by means of the action direction of the spring element which typically is the optical axis of the lens implant. According to the invention, the spring constant is not only dimensioned such that it enables the lens capsule to stretch the spring element and by this enlarges the distance between the viewing elements along the optical axis, but is dimensioned such that the lens capsule is enabled to separate the viewing elements up to a distance which represents a state for near vision. The transition shall preferably be effected solely by means of tensions in the lens capsule. The lens implant lacks of elements for directly or indirectly engaging, in the deployed state, with a contracting ciliary muscle of the eye.

It is believed that the present lens although comprising two spaced apart viewing elements may be closely aligned to the shape and the dimension of the natural lens mass and the lens implant as well as its actuation may be conform to the natural lens and its actuation. With the actuation being the same as with the natural lens, i.e. in particular without the ciliary muscle directly acting on the lens implant, a lens implanted person is not needed to experience, learn and adapt a different way of actuation / accommodation whereas a direct engagement of the ciliary muscle with a clamp of the lens implant may be irritating. In addition, it is believed that whenever the lens mass can be replicated into the synthetic lens implant at its best in shape and dimension, the basement membrane forming the lens capsule will likely better engage with the lens implant for the reason of a better fit and which may better prevent from corrosion and clouding. It is believed that the subcapsular epithelium from which the basement membrane is built will show a better sustainability when engaged with an aligned lens implant which follows the natural lens in the shape and actuation.

For other embodiments of the present aspect of the invention it is referred to the dependent claims.

According to another aspect of the present invention, there is provided a method for manufacturing a lens implant according to any one of the previous embodiments. The lens implant comprises a first viewing element, a second viewing element, and a spring element connected to the viewing elements for varying a distance between the first and the second viewing element along an optical axis of the lens implant for varying the focal length of the lens implant. The lens implant is designed to take a shape suitable for distant vision when the spring element is in its relaxed state. A spring constant of the spring element has a value of less than 550 mN/mm. Specifically, the lens implant is designed to take a shape suitable for near vision when the spring element is in a stretched state such that the distance between the first and the second viewing element in the stretched state of the spring element exceeds the distance between the first and the second viewing element in the relaxed state of the spring element. Hence, the lens implant is designed to require an external stretch force which, upon contraction of the ciliary muscle, originates from the lens capsule and acts on the spring element for stretching the spring element from its relaxed state into its stretched state. Further, the spring constant of the spring element is dimensioned such that, in a deployed state of the lens implant in a lens capsule of an eye, the force produced by the lens capsule transforms the spring element from its relaxed state into the stretched state upon contraction of the ciliary muscle, which stretched state results in a shape of the lens implant representing a lens accommodated to near vision.

The lens implant lacks of elements for directly or indirectly engaging, in the deployed state, with a contracting ciliary muscle of the eye.

According to the manufacturing method, the natural lens is measured. In particular its shape and dimensions are measured by any imaging technique. The data derived from such measurement may be used for forming the lens implant. The spring element is formed with a spring constant such that the spring element is expected to be stretchable by tension forces induced by the lens capsule surrounding the natural lens. Such tension forces may be measured or be estimated. At least one of the two viewing elements is formed with a desired optical power which desired optical power is derived from the measurement.

According to another aspect of the present invention, there is provided a kit for manufacturing a lens implant according to any one of the previous embodiments. The lens implant comprises a first viewing element, a second viewing element, and a spring element connectable to the viewing elements for varying a distance between the first and the second viewing element along an optical axis of the lens implant for varying the focal length of the lens implant. The lens implant is designed to take a shape suitable for distant vision when the spring element is in its relaxed state. A spring constant of the spring element has a value of less than 550 mN/mm. Specifically, the lens implant is designed to take a shape suitable for near vision when the spring element is in a stretched state such that the distance between the first and the second viewing element in the stretched state of the spring element exceeds the distance between the first and the second viewing element in the relaxed state of the spring element. Hence, the lens implant is designed to require an external stretch force which, upon contraction of the ciliary muscle, originates from the lens capsule and acts on the spring element for stretching the spring element from its relaxed state into its stretched state. Further, the spring constant of the spring element is dimensioned such that, in a deployed state of the lens implant in a lens capsule of an eye, the force produced by the lens capsule transforms the spring element from its relaxed state into the stretched state upon contraction of the ciliary muscle, which stretched state results in a shape of the lens implant representing a lens accommodated to near vision. The transition shall preferably be effected solely by means of tensions in the lens capsule. The lens implant lacks of elements for directly or indirectly engaging, in the deployed state, with a contracting ciliary muscle of the eye.

The kit comprises multiple viewing elements with different focal lengths and/or different shapes, and a spring element for holding the viewing elements.

Any embodiments described with respect to the device shall similarly pertain to the method and the kit. Synergetic effects may arise from different combinations of the embodiments although they might not be described in detail.

Further on it shall be noted that all embodiments of the present invention concerning a method might be carried out with the order of the steps as described, nevertheless this has not to be the only essential order of the steps of the method all different orders of orders and combinations of the method steps are herewith described.

### Brief Description of the Drawings

The aspects defined above and further aspects, features and advantages of the present invention can also be derived from the examples of embodiments to be described hereinafter and are explained with reference to examples of embodiments. The invention will be described in more detail hereinafter with reference of examples of embodiments but to which the invention is not limited.
Fig. 1 shows a longitudinal cut of a schematic intraocular lens implant according to an embodiment of the present invention, in Fig 1a in its relaxed state, and in Fig. 1b in its stretched state;
Fig. 2 shows a longitudinal cut of a schematic intraocular lens implant according to an embodiment of the present invention, implanted in a lens capsule, in Fig. 2a in the stretched state of the lens capsule, and in Fig, 2b in the relaxed state of the capsule;
Fig. 3 shows a longitudinal cut of a front portion of the human eye;
Fig. 4 shows a longitudinal cut of a front portion of an eye with an implant according to an embodiment of the present invention in a state accommodated to distant vision; and
Fig. 5 shows a longitudinal cut of a front portion of an eye with an implant according to an embodiment of the present invention in a state accommodated to near vision.

### Modes for Carrying Out the Invention

Similar or relating components in the several figures may be provided with the same reference numerals.

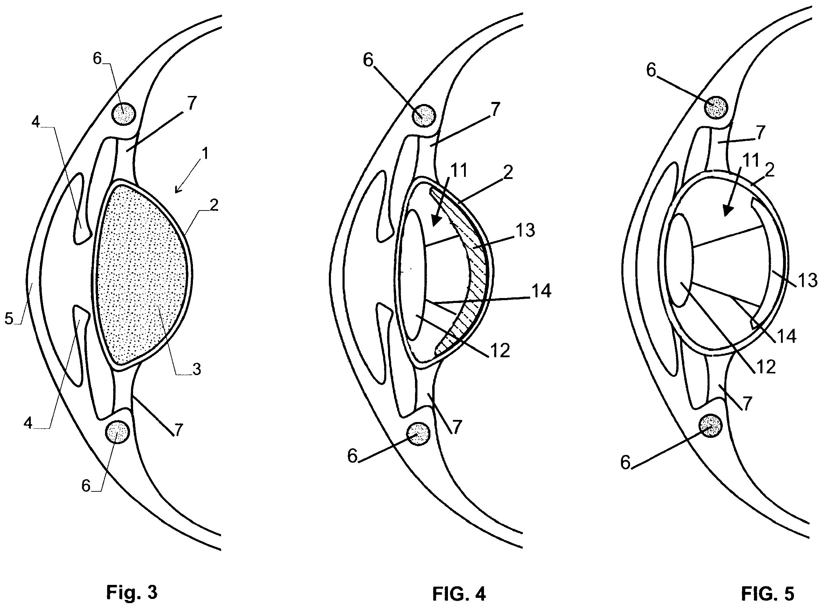

In Figure 3 it is referred to a simplified cross section of a front part of the human eye which comprises a cornea 5, an iris 4 and a lens 1 comprising a lens mass 3 arranged in a lens capsule 2. The lens 1 is connected via zonule fibres 7 to a ciliary muscle 6. The ciliary muscle 6 takes the form of a ring that may contract and relax. A contraction of the ciliary muscle 6 shall lead to accommodation which is understood as the eye focusing to an object in the near vision. Relaxation of the ciliary muscle 6 shall lead to a less accommodated state also referred to as unaccommodated state in which the eye is prepared for distant vision.

In a state in which the lens 1 is adapted for distant vision, the ciliary muscle 6 is relaxed as shown in Figure 3. In such state the zonule fibres 7 are tense and pull the edge of the lens capsule 2 radially outwards such that the lens 1 takes a rather flat shape in view of the drag force generated by the ciliary muscle 6 and transmitted by the zonule fibres 7 to the lens capsule 2. Hence, the lens capsule 2 itself is not in a relaxed state but is radially pulled such that it takes a rather flat shape instead of a spherical shape. Such configuration with a rather flat shape of the lens 1 enables for distant vision for the reason that the lens 1 is not shaped as to provide a focal length in the near field.

When transitioning from distant vision to near vision, the ciliary muscle 6 contracts such that a diameter of the ciliary muscle 6 around the lens 1 decreases. As a result, the tension in the zonule fibres 7 drops and the zonule fibres 7 may only hold the lens 1 but not add any additional radial forces to the lens 1. In such state, i.e. for a lens without the application of any external forces, the lens 1 relaxes from its flat shape and returns to the near spherical shape for near vision in which the focal length of the lens 1 is much smaller than for distant vision.

It was observed that absent any external forces acting on the lens 1 the lens mass 3 takes a rather flat shape suited for distant vision. However, when the lens mass 3 will be encapsulated in the lens capsule 2 it will be deformed and transform from the flat shape into a more spherical shape suited for near vision, again, absent any external forces. The lens capsule contains fibres built during the building of the lens mass Absent any external forces these fibres make the lens capsule to take a shape of lowest energy which results in a form with the smallest surface per volume which presently is a sphere - or better a sphere-like - structure.

Summarizing, absent any external forces applied to the lens capsule the lens mass / lens capsule combination will take a rather spherical form representing a lens suited for near vision. The forces generated by the lens capsule are sufficient for effecting such deformation of the lens mass 3 as the zonule fibres 7 are effete with the cilicary muscle 6 being contracted. And returning to the distant vision, the surface tension paradigm prevailing for the near view will be overridden by zonula fibres 7 pulling the edge of the lens capsule 2 outwards in response to the ciliary muscle 6 relax which makes the zonula fibres 7 become tense. As a result, the lens capsule 2 is radially stretched and takes a rather flat form suitable for distant vision.

A lens implant in the present context is understood as an implant for replacing the lens mass but not the lens capsule. Accordingly, the lens implant is meant to be inserted into the lens capsule.

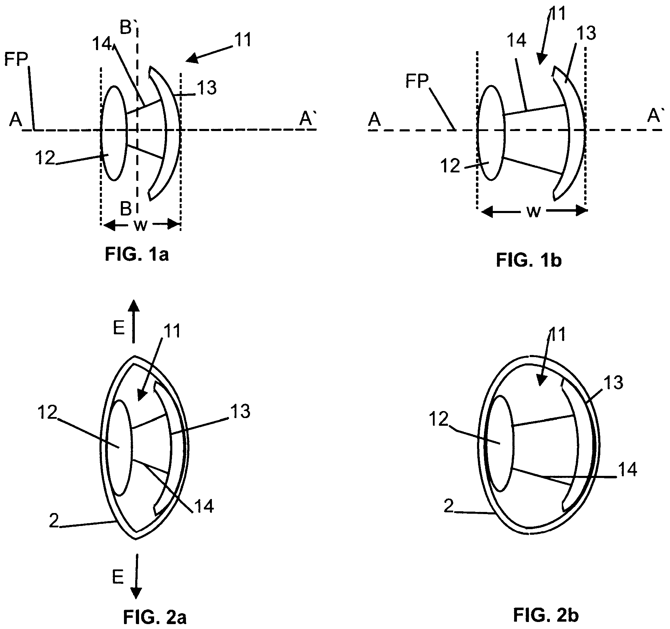

In an embodiment of the present invention Fig. 1 shows a longitudinal cut of a schematic intraocular lens implant 11. The lens implant 11 includes two viewing elements 12 and 13 and a spring element 14 between the viewing elements 12 and 13. The present lens implant 11 is a simplified version as the person skilled may easily comprehend that other shapes of the viewing elements, different forms of spring elements etc. may be encompassed by such lens implant 11, too.

Axis A - A' denotes the optical axis of the lens implant 1. Axis B - B' denotes the longitudinal axis of the lens implant 1. The spring element 14 is connected to both viewing elements 13 and 14 and is arranged such that a distance between the viewing elements 12, 13 along the optical axis can be varied subject to the force applied to the viewing elements 12, 13. A sample focal point on the optical axis is denoted as FP.

In Fig. 1a, the spring element 14 is in its relaxed state, i.e. no external forces are acting on the spring element 14 or the viewing elements 12, 13. Fig. 1a represents a lens implant 1 e.g. after manufacture and prior to implantation. The spring element 14 is dimensioned such that in its relaxed state the viewing elements 12, 13 are spaced from each other at a distance which implements a lens implant 1 focusing in the distance.

In such relaxed state a width w of the lens implant 11 along the optical axis A-A' between outer surfaces of the first and the second viewing element 12, 13 may preferably be between 2.5 mm and 5.5 mm in the relaxed state of the spring element 14, and in a very preferred embodiment be between 3.8 mm and 4.0 mm in the relaxed state of the spring element 14.

In contrast, in Fig. 1b the spring element 14 is in a stretched, extended state, i.e. external forces are applied to the spring element 14 or the viewing elements 12, 13 and make the distance between the viewing elements 12, 13 increase, and in particular exceed the distance between the viewing elements 12, 13 compared to a situation when the spring element 14 is unloaded according to Fig. 1a. Now, the viewing elements 12, 13 are spaced apart at a distance which results in a lens implant 1 focusing to the near, e.g. on focal point FP. The spring element 14 is under tension in this example.

In such stretched state the width w of the lens implant 11 along the optical axis A-A' between outer surfaces of the first and the second viewing element 12, 13 may preferably be between 2.7 mm and 5.7 mm in the stretched state of the spring element 14, and in a very preferred embodiment be between 4.0 mm and 4.2 mm in the stretched state of the spring element 14.

Fig. 2 shows a longitudinal cut of a schematic intraocular lens implant 11 according to an embodiment of the present invention, now implanted in a lens capsule 2. For illustration purposes it is assumed that (except for gravitation, of course) no other forces than the spring force of the spring element 13 and tension forces inherent in the lens capsule 2 are interacting.

In Fig. 2b the lens capsule 2 is in its relaxed state and takes a shape of lowest energy. No external forces are assumed to apply to such implant/capsule combination. It is apparent that the relaxed state of the combination of the lens implant 11 and the lens capsule 2 is not equivalent to the relaxed state of the lens implant 11 on its own. Rather, the lens implant 11 is in its stretched state and is accommodated to the near. The force responsible for transitioning the lens implant 11 from its inherent relaxed state according to Fig. 1a to its stretched state according to Fig. 2b is evoked by the lens capsule 2. The lens capsule 2 - without any external forces applied - is taking a shape of lowest energy which - as far as the spring element 14 of the lens implant 1 is not counteracting - is a spherical-like shape. Tension and in particular surface tension built in the lens capsule 2 is responsible for such transition.

However, if the spring element 14 of the lens implant 1 would have been designed with a very high spring constant such that only for a large force applied the spring element 14 may stretch, the counteracting forces evoked by the lens capsule 2 would not suffice to exceed the spring force and the distance between the viewing elements 12, 13 would not change significantly.

According to the invention, the spring constant of the spring element 14 has a value of less than 550 mN/mm.

In a preferred embodiment, the spring constant of the spring element 14 has a value of less than 20 mN/mm.

In another preferred embodiment, the spring constant of the spring element 14 has a value of more than 2.5 mN/mm.

In another preferred embodiment, the spring constant of the spring element 14 has a value of more than 10 mN/mm.

Any combinations of the above ranges of the spring constant are considered as preferred embodiments: The spring constant may be designed in one of a range between 2.5 mN/mm and 20 mN/mm, a range between 10 mN/mm and 20 mN/mm, a range between 2.5 mN/mm and 550 mN/mm, and a range between 10 mN/mm and 550 mN/mm.

Taking the gravitational field strength into account, the above ranges may be described as one of less than 55 g/mm, more than 0.25 g/mm, more than 1 g/mm, a range between 0.25 g/mm and 55 g/mm, a range between 1 g/mm and 55 g/mm, a range between 0.25 g/mm and 2 g/mm, or a range between 1 g/mm and 2 g/mm.

For this reason the spring constant of the spring element 14 is dimensioned such that a force produced by the lens capsule 2 transforms the spring element 14 from its relaxed state into a stretched state. In other words, in a direction of the optical axis A - A' of the lens implant 11, the forces generated by the lens capsule 2 need to exceed the counteracting force of the spring element 14. In a very preferred embodiment, the force generated by the lens capsule 2 in such direction needs to overcome the spring force by an amount that allows the two viewing elements 12, 13 travelling away from each other until the lens implant 11 is in a condition that allows viewing to the near which is illustrated in Fig. 2b. In a preferred embodiment, a force induced by a mass of the order of g or mg may allow to pull the spring in a mm or sub-mm range.

In Figure 2a, the lens capsule 2 is far from taking its preferred shape of a spherical-like capsule but is rather lengthy and flattened. On the other hand, the lens implant 11 within the lens capsule 2 now is close to its relaxed state which is defined as state where the spring element 14 is in a relaxed state. When the spring element 14 on its own would traverse from a stretched state as shown in Fig. 2b to a relaxed state as shown in Fig. 2a, it would have to overcome the tension exerted by the lens capsule 1. Such tension may be overcome by forces applied to upper edges of the lens capsule 2, as indicted by arrows E. Such forces may be evoked through relaxing of the ciliary muscle 6 which in turn strains the zonule fibres 7.

A lens implant 11 implanted in the eye is schematically illustrated in the longitudinal cut of Fig. 4. The lens capsule 2 encapsulates the lens implant 11. Zonule fibres 7 radially attached to the lens capsule 2 are in a stretched state. A rather flat capsule / implant combination is formed (at least flatter than the spherical shaped body of the lens implant 11 of Fig. 5, wherein the flat capsule / implant combination in Fig. 4 represents a state/shape for distant vision). According to Fig. 4, a relaxing of the ciliary muscle 6 in turn evokes straining the zonule fibres 7 which in turn stretch the lens capsule 2.

Fig. 5 in turn shows a longitudinal cut of the eye of Figur 4, however, in a state accommodated to near vision. Between the states of Fig. 4 and Fig 5, the actor, i.e. the ciliary muscle 6 has contracted in order to accommodate to the near. When the ring like muscle is contracted, the zonule fibres 7 relax and do no longer pull the edges of the lens capsule 2. For this reason, the lens capsule 2 takes the shape of lowest energy which is a spherical like shape to the extent the spring element 14 of the lens implant 11 allows.

Generally, for the present intraocular lens implant it is beneficial that a longitudinal extension of the lens implant along the optical axis in the relaxed state of the spring element is less than a longitudinal extension of the lens implant along the optical axis in the stretched state. This makes the lens implant be suitable for near vision in its excited state rather than to distant vision. In near vision, the focal length as distance between the focal point on the optical axis and the lens implant is less than the focal length in distant vision.

According to the invention, the lens implant lacks of elements to engage with the ciliary muscle of the eye upon contraction of the ciliary muscle. I.e., the lens implant is not directly or indirectly controllable in its shape by a contraction of the ciliary muscle. In other words, the shape of the lens implant will not be affected by a contracting ciliary muscle. According to the invention, the shape of the lens implant is solely affected by forces induced via the two viewing elements. This may include, that protrusions designed to shorten the distance between the viewing elements and the ciliary muscle or zonule fibres for an engagement between the cilicary muscle or the zonule fibres and the protrusions are avoided. Advantageously, the lens implant lacks of elements exceeding a height of the lens capsule in its relaxed state wherein the height is defined along the axis B - B' of Figure 1a. Advantageously, the lens implant lacks of elements projecting above the height of the viewing elements in a direction of a longitudinal axis of the viewing elements. In other word, upper edges of the viewing elements may terminate the lens in a direction of a longitudinal axis of the lens.

The viewing elements and the spring element may advantageously be formed integrally, as a single piece, or alternatively, may be formed from at least the individual viewing elements and the spring element. The viewing elements may comprise a lens with plus power, and a lens with negative power.

Kits for manufacturing a lens implant according to one of the preceding embodiments may be provided such kit comprising multiple viewing elements with different focal lengths and/or different shapes, and at least one spring element for holding the viewing elements. From such kit an individual lens implant may be assembled in an ophthalmic clinic where a patients lens is replaced by the lens implant. From the kit lenses are chosen that match the refractive index, dimension and shape of the patients needs.

The lens implant preferably is adapted to the natural lens of the patient's eye it shall replace. For this reason, the lens implant preferably comprise outside surfaces for being in contact with the lens capsule in an implanted state which outside surfaces take the dimension and form of the specific natural lens mass. In order to get there the natural lens is measured e.g. by computerized imaging which may result in a computerized model of the lens. The lens implant may be formed according the model and as such according to the dimension and shape of the natural lens as far as the individual viewing elements and spring elements allow.

In the previous embodiments, the lens capsule 2 is closed after inserting the lens implant 11. However, precise cuts, for example, a circular cut may be generated at the front portion of the lens capsule 2 by means of laser technology which cut is aligned with the optical axis A - A' such that such cut may not even be closed after inserting the lens implant 11 and may remain open.

While there are shown and described presently preferred embodiments of the invention, it is to be distinctly understood that the invention is not limited thereto but may be otherwise variously embodied and practiced within the scope of the following claims.

## Claims

1. Intraocular lens implant, comprising
a first viewing element (12) and a second viewing element (13),
a spring element (14) connected to the first viewing element (13) and the second viewing element (14) for varying a distance between the first and the second viewing element (12, 13) along an optical axis (A-A') of the lens implant (11) for varying the focal length of the lens implant (11),
wherein the lens implant (11) is designed to take a shape suitable for distant vision when the spring element (14) is in a relaxed state,
wherein the lens implant (11) is designed to take a shape suitable for near vision when the spring element (14) is in a stretched state such that the distance between the first and the second viewing element (12, 13) in the stretched state of the spring element (14) exceeds the distance between the first and the second viewing element (12, 13) in the relaxed state of the spring element (14),
wherein the lens implant (11) is designed to require an external stretch force for stretching the spring element (14) from its relaxed state into its stretched state, wherein the external stretch force originates, in a deployed state of the lens implant (11) in a lens capsule (2) of an eye and upon contraction of a ciliary muscle (6), from the lens capsule (2) acting on the spring element (14),
wherein a spring constant of the spring element (14) is dimensioned such that, in the deployed state and upon contraction of the ciliary muscle (6), the force produced by the lens capsule (2) transforms the spring element (14) from its relaxed state into the stretched state, which stretched state results in a shape of the lens implant (11) representing a lens accommodated to near vision,
wherein the spring constant of the spring element (14) has a value of less than 550 mN/mm,
wherein the lens implant (11) lacks elements for directly or indirectly engaging, in the deployed state, with the contracting ciliary muscle (6) of the eye, and
wherein the lens implant (11) is designed for solely being controlled by tension forces induced by the lens capsule (2) via the viewing elements (12, 13).

2. Intraocular lens implant according to claim 1,
wherein the spring constant of the spring element (14) has a value of less than 20 mN/mm.

3. Intraocular lens implant according to any one of the preceding claims,
wherein the spring constant of the spring element (14) has a value of more than 2.5 mN/mm.

4. Intraocular lens implant according to any one of the preceding claims,
wherein a width (w) of the lens implant (11) along the optical axis (A-A') between outer surfaces of the first and the second viewing element (12, 13) is between 2.5 mm and 5.5 mm in the relaxed state of the spring element (14).

5. Intraocular lens implant according to any one of the preceding claims,
wherein a width (w) of the lens implant (11) along the optical axis (A-A') between outer surfaces of the first and the second viewing element (12, 13) is between 2.7 mm and 5.7 mm in the stretched state of the spring element (14).

6. Lens implant according to any one of the preceding claims, wherein a longitudinal extension of the lens implant (11) along the optical axis (A-A') in the relaxed state of the spring element (14) is less than a longitudinal extension of the lens implant (11) along the optical axis (A-A') in the stretched state.

7. Lens implant according to any one of the preceding claims, wherein upper edges of the viewing elements (12, 13) terminate the lens implant (11) in a direction of a longitudinal axis (B-B') of the lens implant (11) .

8. Lens implant according to any one of the preceding claims, wherein the viewing elements (12, 13) and the spring element (14) are formed integrally.

9. Lens implant according to any one of the preceding claims, wherein the first viewing element (12) is a lens with a plus power, and the second viewing element (13) is a lens with a negative power.

10. Method for manufacturing a lens implant according to one of the preceding claims, the lens implant (11) comprising
a first viewing element (12) and a second viewing element (13),
a spring element (14) connected to the first viewing element (13) and the second viewing element (14) for varying a distance between the first and the second viewing element (12, 13) along an optical axis (A-A') of the lens implant (11) for varying the focal length of the lens implant (11),
wherein the lens implant (11) is designed to take a shape suitable for distant vision when the spring element (14) is in a relaxed state,
wherein the lens implant (11) is designed to take a shape suitable for near vision when the spring element (14) is in a stretched state such that the distance between the first and the second viewing element (12, 13) in the stretched state of the spring element (14) exceeds the distance between the first and the second viewing element (12, 13) in the relaxed state of the spring element (14),
wherein the lens implant (11) is designed to require an external stretch force for stretching the spring element (14) from its relaxed state into its stretched state, wherein the external stretch force originates, in a deployed state of the lens implant (11) in a lens capsule (2) of an eye and upon contraction of a ciliary muscle (6), from the lens capsule (2) acting on the spring element (14),
wherein a spring constant of the spring element (14) is dimensioned such that, in the deployed state and upon contraction of the ciliary muscle (6), the force produced by the lens capsule (2) transforms the spring element (14) from its relaxed state into the stretched state, which stretched state results in a shape of the lens implant (11) representing a lens accommodated to near vision,
wherein the spring constant of the spring element (14) has a value of less than 550 mN/mm,
wherein the lens implant (11) lacks elements for directly or indirectly engaging, in the deployed state, with the contracting ciliary muscle (6) of the eye,
wherein the lens implant (11) is designed for solely being controlled by tension forces induced by the lens capsule (2) via the viewing elements (12, 13), and
wherein the method comprises steps of
- measuring the natural lens the lens implant (11) shall replace by an imaging technique,
- forming the spring element (14) with a spring constant such that the spring element is expected to be stretchable by the tension forces induced by the lens capsule surrounding the natural lens, and
- forming at least one of the two viewing elements (12, 13) according to an optical power derived from the measurement.

11. Kit for manufacturing a lens implant according to one of the claims 1 to 7 or claim 9, the lens implant (11) comprising
a first viewing element (12),
a second viewing element (13), and
a spring element (14) connectable to the first viewing element (13) and the second viewing element (14) for varying a distance between the first and the second viewing element (12, 13) along an optical axis (A-A') of the lens implant (11) for varying the focal length of the lens implant (11),
wherein the lens implant (11) is designed to take a shape suitable for distant vision when the spring element (14) is in a relaxed state, and
wherein the lens implant (11) is designed to take a shape suitable for near vision when the spring element (14) is in a stretched state such that the distance between the first and the second viewing element (12, 13) in the stretched state of the spring element (14) exceeds the distance between the first and the second viewing element (12, 13) in the relaxed state of the spring element (14),
wherein the lens implant (11) is designed to require an external stretch force for stretching the spring element (14) from its relaxed state into its stretched state, wherein the external stretch force originates, in a deployed state of the lens implant (11) in a lens capsule (2) of an eye and upon contraction of a ciliary muscle (6), from the lens capsule (2) acting on the spring element (14),
wherein a spring constant of the spring element (14) is dimensioned such that, in the deployed state and upon contraction of the ciliary muscle (6), the force produced by the lens capsule (2) transforms the spring element (14) from its relaxed state into the stretched state, which stretched state results in a shape of the lens implant (11) representing a lens accommodated to near vision,
wherein a spring constant of the spring element (14) has a value of less than 550 mN/mm,
wherein the lens implant (11) lacks elements for directly or indirectly engaging, in the deployed state, with the contracting ciliary muscle (6) of the eye,
wherein the lens implant (11) is designed for solely being controlled by tension forces induced by the lens capsule (2) via the viewing elements (12, 13), and
wherein the kit for manufacturing the lens implant (11) comprises multiple viewing elements (12, 13) with different focal lengths and/or different shapes, and a spring element (14) for holding the viewing elements (12, 13).

## Patentansprüche

1. Intraokulares Linsenimplantat, umfassend
ein erstes Linsenelement (12) und ein zweites Linsenelement (13),
ein Federelement (14), das mit dem ersten Linsenelement (13) und dem zweiten Linsenelement (14) verbunden ist, um einen Abstand zwischen dem ersten und dem zweiten Linsenelement (12, 13) entlang einer optischen Achse (A-A') des Linsenimplantats (11) zu verändern, um die Brennweite des Linsenimplantats (11) zu verändern,
wobei das Linsenimplantat (11) so ausgestaltet ist, dass es eine für die Fernsicht geeignete Form annimmt, wenn sich das Federelement (14) in einem entspannten Zustand befindet,
wobei das Linsenimplantat (11) so ausgestaltet ist, dass es eine für die Nahsicht geeignete Form annimmt, wenn sich das Federelement (14) in einem gestreckten Zustand befindet, so dass der Abstand zwischen dem ersten und dem zweiten Linsenelement (12, 13) im gestreckten Zustand des Federelements (14) den Abstand zwischen dem ersten und dem zweiten Linsenelement (12, 13) im entspannten Zustand des Federelements (14) übersteigt,
wobei das Linsenimplantat (11) so ausgestaltet ist, dass es eine externe Streckkraft benötigt, um das Federelement (14) von seinem entspannten Zustand in seinen gestreckten Zustand zu strecken, wobei die externe Streckkraft in einem eingesetzten Zustand des Linsenimplantats (11) in einer Linsenkapsel (2) eines Auges und bei Kontraktion eines Ziliarmuskels (6) von der Linsenkapsel (2) ausgeht, die auf das Federelement (14) wirkt,
wobei eine Federkonstante des Federelements (14) so bemessen ist, dass im eingesetzten Zustand und bei Kontraktion des Ziliarmuskels (6) die von der Linsenkapsel (2) erzeugte Kraft das Federelement (14) von seinem entspannten Zustand in den gestreckten Zustand überführt, wobei der gestreckte Zustand zu einer Form des Linsenimplantats (11) führt, die eine Linse darstellt, die für die Nahsicht akkommodiert ist,
wobei die Federkonstante des Federelements (14) einen Wert von weniger als 550 mN/mm hat,
wobei das Linsenimplantat (11) keine Elemente aufweist, die im eingesetzten Zustand direkt oder indirekt in den kontrahierenden Ziliarmuskel (6) des Auges eingreifen, und
wobei das Linsenimplantat (11) so ausgestaltet ist, dass es ausschliesslich durch Zugkräfte gesteuert wird, die von der Linsenkapsel (2) über die Linsenelemente (12, 13) eingeleitet werden.

2. Intraokulares Linsenimplantat nach Anspruch 1,
wobei die Federkonstante des Federelements (14) einen Wert von weniger als 20 mN/mm hat.

3. Intraokulares Linsenimplantat nach einem der vorhergehenden Ansprüche,
wobei die Federkonstante des Federelements (14) einen Wert von mehr als 2.5 mN/mm hat.

4. Intraokulares Linsenimplantat nach einem der vorhergehenden Ansprüche,
wobei eine Breite (w) des Linsenimplantats (11) entlang der optischen Achse (A-A') zwischen Aussenflächen des ersten und des zweiten Linsenelements (12, 13) im entspannten Zustand des Federelements (14) zwischen 2.5 mm und 5.5 mm beträgt.

5. Intraokulares Linsenimplantat nach einem der vorhergehenden Ansprüche,
wobei eine Breite (w) des Linsenimplantats (11) entlang der optischen Achse (A-A') zwischen Aussenflächen des ersten und des zweiten Linsenelements (12, 13) im gestreckten Zustand des Federelements (14) zwischen 2.7 mm und 5.7 mm beträgt.

6. Linsenimplantat nach einem der vorhergehenden Ansprüche, wobei eine Längsausdehnung des Linsenimplantats (11) entlang der optischen Achse (A-A') im entspannten Zustand des Federelements (14) geringer ist als eine Längsausdehnung des Linsenimplantats (11) entlang der optischen Achse (A-A') im gestreckten Zustand.

7. Linsenimplantat nach einem der vorhergehenden Ansprüche, wobei obere Kanten der Linsenelemente (12, 13) das Linsenimplantat (11) in einer Richtung einer Hochachse (B-B') des Linsenimplantats (11) abschliessen.

8. Linsenimplantat nach einem der vorhergehenden Ansprüche, wobei die Linsenelemente (12, 13) und das Federelement (14) einstückig ausgebildet sind.

9. Linsenimplantat nach einem der vorhergehenden Ansprüche, wobei das erste Linsenelement (12) eine Linse mit einer positiven Brechkraft ist und das zweite Linsenelement (13) eine Linse mit einer negativen Brechkraft ist.

10. Verfahren zur Herstellung eines Linsenimplantats nach einem der vorhergehenden Ansprüche, wobei das Linsenimplantat (11) umfasst
ein erstes Linsenelement (12) und ein zweites Linsenelement (13),
ein Federelement (14), das mit dem ersten Linsenelement (13) und dem zweiten Linsenelement (14) verbunden ist, um einen Abstand zwischen dem ersten und dem zweiten Linsenelement (12, 13) entlang einer optischen Achse (A-A') des Linsenimplantats (11) zu verändern, um die Brennweite des Linsenimplantats (11) zu verändern,
wobei das Linsenimplantat (11) so ausgestaltet ist, dass es eine für die Fernsicht geeignete Form annimmt, wenn sich das Federelement (14) in einem entspannten Zustand befindet,
wobei das Linsenimplantat (11) so ausgestaltet ist, dass es eine für die Nahsicht geeignete Form annimmt, wenn sich das Federelement (14) in einem gestreckten Zustand befindet, so dass der Abstand zwischen dem ersten und dem zweiten Linsenelement (12, 13) im gestreckten Zustand des Federelements (14) den Abstand zwischen dem ersten und dem zweiten Linsenelement (12, 13) im entspannten Zustand des Federelements (14) übersteigt,
wobei das Linsenimplantat (11) so ausgestaltet ist, dass es eine externe Streckkraft benötigt, um das Federelement (14) von seinem entspannten Zustand in seinen gestreckten Zustand zu strecken, wobei die externe Streckkraft in einem eingesetzten Zustand des Linsenimplantats (11) in einer Linsenkapsel (2) eines Auges und bei Kontraktion eines Ziliarmuskels (6) von der Linsenkapsel (2) ausgeht, die auf das Federelement (14) wirkt,
wobei eine Federkonstante des Federelements (14) so bemessen ist, dass im eingesetzten Zustand und bei Kontraktion des Ziliarmuskels (6) die von der Linsenkapsel (2) erzeugte Kraft das Federelement (14) von seinem entspannten Zustand in den gestreckten Zustand überführt, wobei der gestreckte Zustand zu einer Form des Linsenimplantats (11) führt, die eine Linse darstellt, die für die Nahsicht akkommodiert ist,
wobei die Federkonstante des Federelements (14) einen Wert von weniger als 550 mN/mm hat,
wobei das Linsenimplantat (11) keine Elemente aufweist, die im eingesetzten Zustand direkt oder indirekt in den kontrahierenden Ziliarmuskel (6) des Auges eingreifen,
wobei das Linsenimplantat (11) so ausgestaltet ist, dass es ausschliesslich durch Zugkräfte gesteuert wird, die von der Linsenkapsel (2) über die Linsenelemente (12, 13) eingeleitet werden, und
wobei das Verfahren die folgenden Schritte umfasst
- Messung, durch ein bildgebendes Verfahren, der natürlichen Linse, die das Linsenimplantat (11) ersetzen soll,
- Ausbilden des Federelements (14) mit einer solchen Federkonstante, dass zu erwarten ist, dass das Federelement durch die von der die natürliche Linse umgebenden Linsenkapsel eingeleiteten Zugkräfte streckbar ist, und
- Ausbilden mindestens eines der beiden Linsenelemente (12, 13) entsprechend einer aus der Messung abgeleiteten optischen Brechkraft.

11. Bausatz zur Herstellung eines Linsenimplantats nach einem der Ansprüche 1 bis 7 oder Anspruch 9, wobei das Linsenimplantat (11) umfasst
ein erstes Linsenelement (12),
ein zweites Linsenelement (13) und
ein Federelement (14), das mit dem ersten Linsenelement (13) und dem zweiten Linsenelement (14) verbunden werden kann, um einen Abstand zwischen dem ersten und dem zweiten Linsenelement (12, 13) entlang einer optischen Achse (A-A') des Linsenimplantats (11) zu verändern, um die Brennweite des Linsenimplantats (11) zu verändern,
wobei das Linsenimplantat (11) so ausgestaltet ist, dass es eine für die Fernsicht geeignete Form annimmt, wenn sich das Federelement (14) in einem entspannten Zustand befindet, und
wobei das Linsenimplantat (11) so ausgestaltet ist, dass es eine für die Nahsicht geeignete Form annimmt, wenn sich das Federelement (14) in einem gestreckten Zustand befindet, so dass der Abstand zwischen dem ersten und dem zweiten Linsenelement (12, 13) im gestreckten Zustand des Federelements (14) den Abstand zwischen dem ersten und dem zweiten Linsenelement (12, 13) im entspannten Zustand des Federelements (14) übersteigt,
wobei das Linsenimplantat (11) so ausgestaltet ist, dass es eine externe Streckkraft benötigt, um das Federelement (14) von seinem entspannten Zustand in seinen gestreckten Zustand zu strecken, wobei die externe Streckkraft in einem eingesetzten Zustand des Linsenimplantats (11) in einer Linsenkapsel (2) eines Auges und bei Kontraktion eines Ziliarmuskels (6) von der Linsenkapsel (2) ausgeht, die auf das Federelement (14) wirkt,
wobei eine Federkonstante des Federelements (14) so bemessen ist, dass im eingesetzten Zustand und bei Kontraktion des Ziliarmuskels (6) die von der Linsenkapsel (2) erzeugte Kraft das Federelement (14) von seinem entspannten Zustand in den gestreckten Zustand überführt, wobei der gestreckte Zustand zu einer Form des Linsenimplantats (11) führt, die eine Linse darstellt, die für die Nahsicht akkommodiert ist,
wobei eine Federkonstante des Federelements (14) einen Wert von weniger als 550 mN/mm hat,
wobei das Linsenimplantat (11) keine Elemente aufweist, die im eingesetzten Zustand direkt oder indirekt in den kontrahierenden Ziliarmuskel (6) des Auges eingreifen,
wobei das Linsenimplantat (11) so ausgestaltet ist, dass es ausschliesslich durch Zugkräfte gesteuert wird, die von der Linsenkapsel (2) über die Linsenelemente (12, 13) eingeleitet werden, und
wobei der Bausatz zur Herstellung des Linsenimplantats (11) mehrere Linsenelemente (12, 13) mit unterschiedlichen Brennweiten und/oder unterschiedlichen Formen und ein Federelement (14) zum Halten der Linsenelemente (12, 13) umfasst.

## Revendications

1. Implant de lentille intraoculaire, comprenant
un premier élément de vision (12) et un deuxième élément de vision (13),
un élément ressort (14) connecté au premier élément de vision (13) et au deuxième élément de vision (14) pour varier une distance entre le premier et le deuxième élément de vision (12, 13) le long d'un axe optique (AA') de l'implant de lentille (11) pour varier la distance focale de l'implant de lentille (11),
dans lequel l'implant de lentille (11) est conçu pour prendre une forme appropriée pour la vision de loin lorsque l'élément ressort (14) est dans un état relâché,
dans lequel l'implant de lentille (11) est conçu pour prendre une forme appropriée pour la vision de près lorsque l'élément ressort (14) est dans un état étiré de telle sorte que la distance entre le premier et le deuxième élément de vision (12, 13) dans l'état étiré de l'élément ressort (14) dépasse la distance entre le premier et le deuxième élément de vision (12, 13) dans l'état relâché de l'élément ressort (14),
dans lequel l'implant de lentille (11) est conçu pour nécessiter une force d'étirement externe pour étirer l'élément ressort (14) de son état relâché à son état étiré, dans lequel la force d'étirement externe provient, dans un état déployé de l'implant de lentille ( 11) dans une capsule du cristallin (2) d'un œil et lors de la contraction d'un muscle ciliaire (6), de la capsule du cristallin (2) agissant sur l'élément ressort (14),
dans lequel une constante d'élasticité de l'élément ressort (14) est dimensionnée de telle sorte que, dans l'état déployé et lors de la contraction du muscle ciliaire (6), la force produite par la capsule du cristallin (2) transforme l'élément ressort (14) de son état relâché à l'état étiré, cet état étiré ayant pour résultat une forme de l'implant de lentille (11) représentant une lentille adaptée à la vision de près,
dans lequel la constante d'élasticité de l'élément ressort (14) a une valeur inférieure à 550 mN/mm,
dans lequel l'implant de lentille (11) est dépourvu d'éléments pour interagir directement ou indirectement, dans l'état déployé, avec le muscle ciliaire de contraction (6) de l'œil, et
dans lequel l'implant de lentille (11) est conçu pour être uniquement contrôlé par des forces de tension induites par la capsule de cristallin (2) via les éléments de vision (12, 13).

2. Implant de lentille intraoculaire selon la revendication 1,
dans lequel la constante d'élasticité de l'élément ressort (14) a une valeur inférieure à 20 mN/mm.

3. Implant de lentille intraoculaire selon l'une quelconque des revendications précédentes,
dans lequel la constante d'élasticité de l'élément ressort (14) a une valeur supérieure à 2,5 mN/mm.

4. Implant de lentille intraoculaire selon l'une quelconque des revendications précédentes,
dans lequel une largeur (w) de l'implant de lentille (11) le long de l'axe optique (AA') entre les surfaces externes du premier et du deuxième élément de vision (12, 13) est comprise entre 2,5 mm et 5,5 mm dans l'état relâché de l'élément ressort (14).

5. Implant de lentille intraoculaire selon l'une quelconque des revendications précédentes,
dans lequel une largeur (w) de l'implant de lentille (11) le long de l'axe optique (AA') entre les surfaces externes du premier et du deuxième élément de vision (12, 13) est comprise entre 2,7 mm et 5,7 mm dans l'état étiré de l'élément ressort (14).

6. Implant de lentille selon l'une quelconque des revendications précédentes, dans lequel une extension longitudinale de l'implant de lentille (11) le long de l'axe optique (AA') dans l'état relâché de l'élément ressort (14) est inférieure à une extension longitudinale de l'implant de lentille (11) le long de l'axe optique (AA') dans l'état étiré.

7. Implant de lentille selon l'une quelconque des revendications précédentes, dans lequel les bords supérieurs des éléments de vision (12, 13) terminent l'implant de lentille (11) dans une direction d'un axe longitudinal (BB') de l'implant de lentille (11).

8. Implant de lentille selon l'une quelconque des revendications précédentes, dans lequel les éléments de vision (12, 13) et l'élément ressort (14) sont formés d'une seule pièce.

9. Implant de lentille selon l'une quelconque des revendications précédentes, dans lequel le premier élément de vision (12) est une lentille à puissance positive, et le deuxième élément de vision (13) est une lentille à puissance négative.

10. Procédé de fabrication d'un implant de lentille selon l'une des revendications précédentes, l'implant de lentille (11) comprenant
un premier élément de vision (12) et un deuxième élément de vision (13),
un élément ressort (14) connecté au premier élément de vision (13) et au deuxième élément de vision (14) pour varier une distance entre le premier et le deuxième élément de vision (12, 13) le long d'un axe optique (AA') de l'implant de lentille (11) pour varier la distance focale de l'implant de lentille (11),
dans lequel l'implant de lentille (11) est conçu pour prendre une forme appropriée pour la vision de loin lorsque l'élément ressort (14) est dans un état relâché,
dans lequel l'implant de lentille (11) est conçu pour prendre une forme appropriée pour la vision de près lorsque l'élément ressort (14) est dans un état étiré de telle sorte que la distance entre le premier et le deuxième élément de vision (12, 13) dans l'état étiré de l'élément ressort (14) dépasse la distance entre le premier et le deuxième élément de vision (12, 13) dans l'état relâché de l'élément ressort (14),
dans lequel l'implant de lentille (11) est conçu pour nécessiter une force d'étirement externe pour étirer l'élément ressort (14) de son état relâché à son état étiré, dans lequel la force d'étirement externe provient, dans un état déployé de l'implant de lentille ( 11) dans une capsule du cristallin (2) d'un œil et lors de la contraction d'un muscle ciliaire (6), de la capsule du cristallin (2) agissant sur l'élément ressort (14),
dans lequel une constante d'élasticité de l'élément ressort (14) est dimensionnée de telle sorte que, dans l'état déployé et lors de la contraction du muscle ciliaire (6), la force produite par la capsule du cristallin (2) transforme l'élément ressort (14) de son état relâché à l'état étiré, cet état étiré ayant pour résultat une forme de l'implant de lentille (11) représentant une lentille adaptée à la vision de près,
dans lequel la constante d'élasticité de l'élément ressort (14) a une valeur inférieure à 550 mN/mm,
dans lequel l'implant de lentille (11) est dépourvu d'éléments pour interagir directement ou indirectement, dans l'état déployé, avec le muscle ciliaire de contraction (6) de l'œil,
dans lequel l'implant de lentille (11) est conçu pour être uniquement contrôlé par des forces de tension induites par la capsule de cristallin (2) via les éléments de vision (12, 13), et
dans lequel le procédé comprend les étapes de
- mesure du cristallin naturel que l'implant de lentille (11) doit remplacer par une technique d'imagerie,
- formation de l'élément ressort (14) avec une constante d'élasticité telle que l'élément ressort est censé être étirable par les forces de tension induites par la capsule du cristallin entourant le cristallin naturel, et
- formation d'au moins l'un des deux éléments de vision (12, 13) en fonction d'une puissance optique déduite de la mesure.

11. Kit de fabrication d'un implant de lentille selon l'une des revendications 1 à 7 ou la revendication 9, l'implant de lentille (11) comprenant
un premier élément de vision (12),
un deuxième élément de vision (13), et
un élément ressort (14) pouvant être connecté au premier élément de vision (13) et au deuxième élément de vision (14) pour varier une distance entre le premier et le deuxième élément de vision (12, 13) le long d'un axe optique (AA') de l'implant de lentille (11) pour varier la distance focale de l'implant de lentille (11),
dans lequel l'implant de lentille (11) est conçu pour prendre une forme appropriée pour la vision de loin lorsque l'élément ressort (14) est dans un état relâché, et
dans lequel l'implant de lentille (11) est conçu pour prendre une forme appropriée pour la vision de près lorsque l'élément ressort (14) est dans un état étiré de telle sorte que la distance entre le premier et le deuxième élément de vision (12, 13) dans l'état étiré de l'élément ressort (14) dépasse la distance entre le premier et le deuxième élément de vision (12, 13) dans l'état relâché de l'élément ressort (14),
dans lequel l'implant de lentille (11) est conçu pour nécessiter une force d'étirement externe pour étirer l'élément ressort (14) de son état relâché à son état étiré, dans lequel la force d'étirement externe provient, dans un état déployé de l'implant de lentille (11) dans une capsule du cristallin (2) d'un œil et lors de la contraction d'un muscle ciliaire (6), de la capsule du cristallin (2) agissant sur l'élément ressort (14),
dans lequel une constante d'élasticité de l'élément ressort (14) est dimensionnée de telle sorte que, dans l'état déployé et lors de la contraction du muscle ciliaire (6), la force produite par la capsule du cristallin (2) transforme l'élément ressort (14) de son état relâché à l'état étiré, cet état étiré ayant pour résultat une forme de l'implant de lentille (11) représentant une lentille adaptée à la vision de près,
dans lequel une constante d'élasticité de l'élément ressort (14) a une valeur inférieure à 550 mN/mm,
dans lequel l'implant de lentille (11) est dépourvu d'éléments pour interagir directement ou indirectement, dans l'état déployé, avec le muscle ciliaire de contraction (6) de l'œil,
dans lequel l'implant de lentille (11) est conçu pour être uniquement contrôlé par des forces de tension induites par la capsule de cristallin (2) via les éléments de vision (12, 13), et
dans lequel le kit de fabrication de l'implant de lentille (11) comprend plusieurs éléments de vision (12, 13) avec différentes distances focales et/ou différentes formes, et un élément ressort (14) pour maintenir les éléments de vision (12, 13).
